(19) Europäisches Patentamt
European Patent Office
Office européen des brevets

(11) Veröffentlichungsnummer: **0 018 315**
**A1**

(12) # EUROPÄISCHE PATENTANMELDUNG

(21) Anmeldenummer: 80810052.3

(22) Anmeldetag: 11.02.80

(51) Int. Cl.³: **C 07 C 33/46**, C 07 C 33/48, C 07 C 47/55, C 07 C 121/36, C 07 C 29/00, C 07 C 29/14, C 07 C 29/42, A 01 N 53/00

(30) Priorität: 04.04.79 CH 3134/79
23.01.80 CH 541/80

(43) Veröffentlichungstag der Anmeldung: 29.10.80
Patentblatt 80/22

(84) Benannte Vertragsstaaten: CH DE FR GB IT NL

(71) Anmelder: CIBA-GEIGY AG, Patentabteilung Postfach, CH-4002 Basel (CH)

(72) Erfinder: Gsell, Laurenz, Dr., Maiengasse 56, CH-4056 Basel (CH)
Erfinder: Ackermann, Peter, Dr., Reichensteinerstrasse 12, CH-4153 Reinach (CH)
Erfinder: Drabek, Jozef, Dr., Benkenstrasse 12, CH-4104 Oberwil (CH)
Erfinder: Farooq, Saleem, Dr., Im Schaiengarten 2, CH-4107 Ettingen (CH)
Erfinder: Wehrli, Rudolf, Dr., Dianastrasse 2, CH-4310 Rheinfelden (CH)

(54) **3-(Fluorbenzyl)-benzylalkohole, Verfahren zu ihrer Herstellung, ihre Verwendung als Zwischenprodukte zur Herstellung von Insektiziden und als Ausgangsmaterialien verwendete 3-(Fluorbenzyl)-benzylaldehyde.**

(57) 3(Fluorbenzyl)-benzylalkohole der Formel

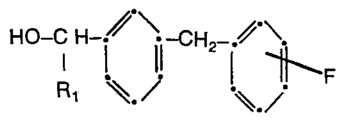

(I)

worin $R_1$ Wasserstoff, Cyano, Äthinyl oder Propinyl bedeutet, Verfahren zu ihrer Herstellung und ihre Verwendung als Zwischenprodukte zur Synthese von Verbindungen, welche sich zur Schädlingsbekämpfung eignen.

EP 0 018 315 A1

0018315



CIBA-GEIGY AG
Basel (Schweiz) 5-12292/1+2

**BEZEICHNUNG GEÄNDERT**
**siehe Titelseite**

3-(Fluorbenzyl)-benzylalkohole, Verfahren zu ihrer Herstellung, ihre Verwendung als Zwischenprodukte und als Ausgangsmaterialien verwendete 3-(fluorbenzyl)-benzylaldehyde.

Die vorliegende Erfindung betrifft 3-(Fluorbenzyl)-benzylalkohole, Verfahren zu ihrer Herstellung und ihre Verwendung als Zwischenprodukte zur Synthese von Verbindungen, welche sich zur Schädlingsbekämpfung eignen. Die 3(Fluorbenzyl)-benzylalkohole haben die Formel

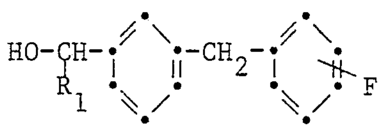

(I)

worin $R_1$ Wasserstoff, Cyano, Aethinyl oder Propinyl bedeutet.

Von besonderem Interesse sind 3(4'-Fluorbenzyl)-benzylalkohole worin $R_1$ die gleiche Bedeutung wie für die Formel I hat.

Die Verbindungen der Formel I können analog bekannten Methoden z.B. wie folgt hergestellt werden:

1) 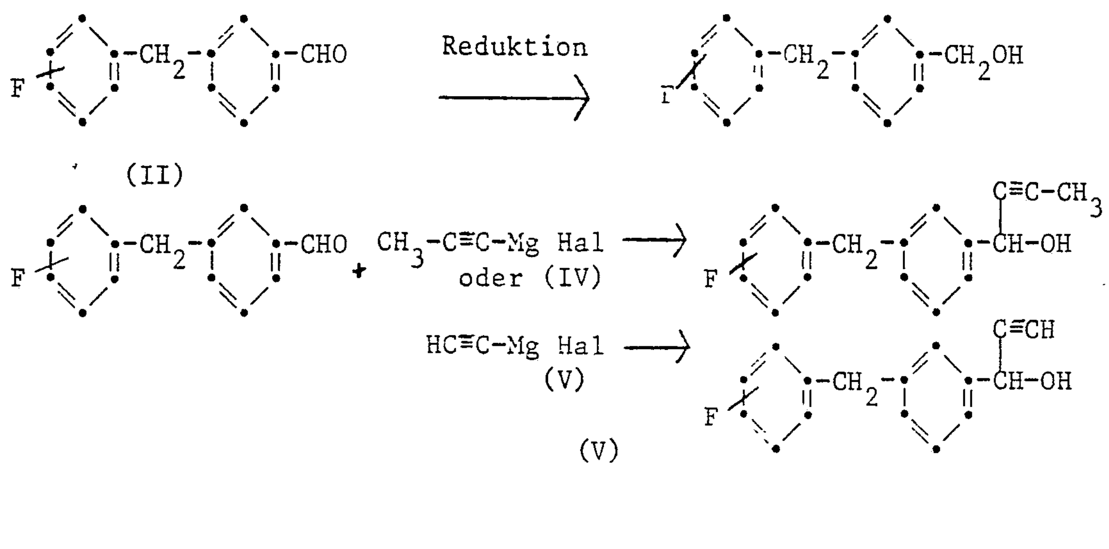

3) 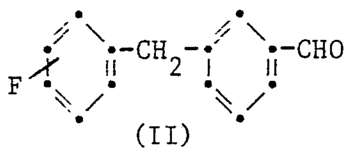 + HCN 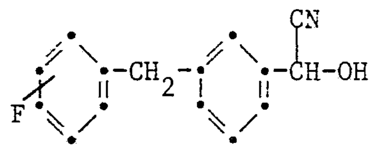

(II)

In den Formeln IV und V steht Hal für ein Halogenatom, insbesondere für Chlor oder Brom. Die Verfahren werden bei einer Reaktionstemperatur von -10 bis 100°C, vorzugsweise bei 0 bis 80°C, im allgemeinen bei Normaldruck und in einem inerten Lösungs- oder Verdünnungsmittel durchgeführt. Als Lösungs- oder Verdünnungsmittel eignen sich vor allem Aether wie Diäthyläther, Tetrahydrofuran oder Dioxan für das zweite Verfahren, während für die Verfahren eins und drei Alkohole wie Methanol oder Aethanol eingesetzt werden können.

Die Verbindungen der Formel I eignen sich als Zwischenprodukte für die Herstellung von Verbindungen (vgl. Bsp. 1b), welche sich zur Bekämpfung von Schädlingen, insbesondere von Insekten und Vertretern der Ordnung Akarina, eignen.

Die verbindung der Formel II sind neu und können wie folgt hergestellt werden:

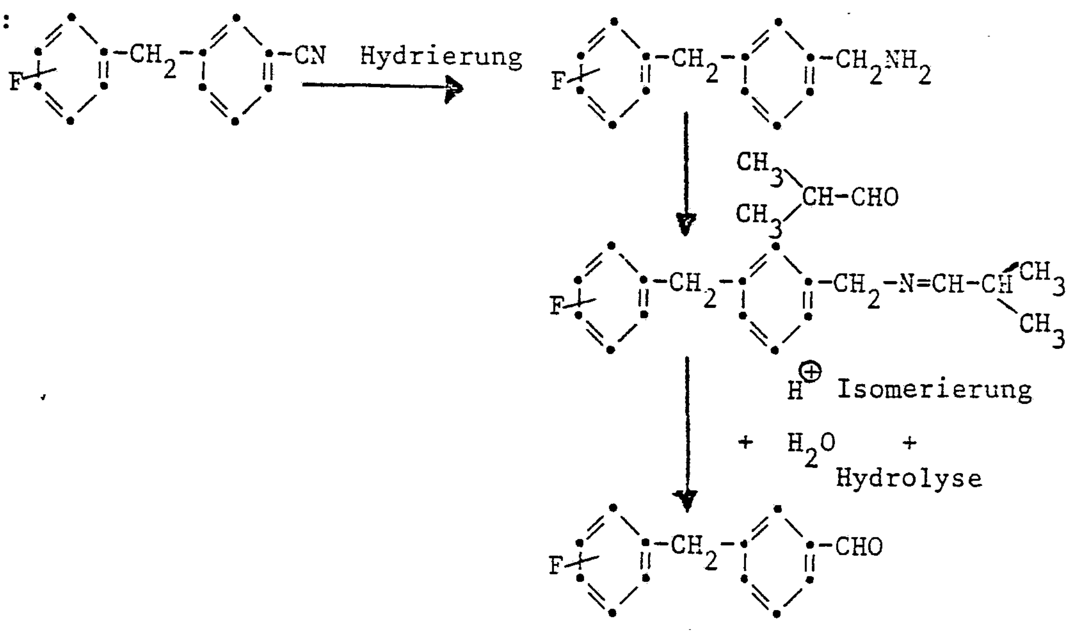


(II)

Beispiel 1: a) Herstellung von 3(4-Fluorbenzyl)-benzylalkohol

28,8 g 3(4-Fluorbenzyl)-benzaldehyd in 200 ml Methanol werden bei 0 bis 5°C innert einer Stunde mit 1,3 g $NaBH_4$ portionenweise versetzt. Nach einer weiteren Stunde wird das Methanol entfernt, mit Wasser versetzt und mit Methylenchlorid extrahiert.

Man erhält die Verbindung der Formel

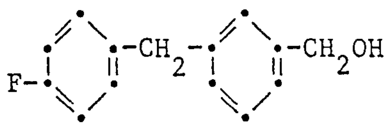

als farbloses Oel das im NMR-Spektrum folgende Signale zeigt:

(60 $MH_Z$) (TMS)

$\delta$ = 3,2 ppm 1 H (S)

$\delta$ = 3,85 ppm 2 H (S)

$\delta$ = 4,45 ppm 2 H (S)

$\delta$ = 7.1 ppm 8 H (m)

b) Herstellung von α-Cyano-3(4-Fluorbenzyl)-benzylalkohol

21,8 g 3(4-Fluorbenzyl)-benzaldehyd in 200 ml Aethanol, 20 ml Wasser und 9,8 g Natriumcyanid werden innerhalb von 30 Minuten mit 15 g Essigsäure versetzt und über Nacht bei 20°C ausgerührt. Nach der teilweisen Entfernung des Alkohols wird 400 ml Aether zugegeben und mit 3 Portionen Wasser gewaschen. Man erhält die Verbindung der Formel

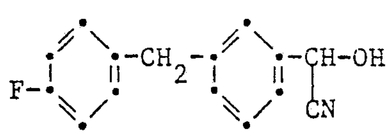

nach dem Trocknen mit Natriumsulfat und dem Entfernen der Lösungsmittel als farbloses Oel mit einer Refraktion von $n_D^{20°}$ = 1,5655

BAD ORIGINAL

Herstellung von α-Cyclopropyl-(4-chlorphenyl)-essigsäure-α-cyano-3-(4-fluorbenzyl)-benzylester

3,63 g α-Cyano-3-(4-fluorbenzyl)-benzylalkohol, 3,43 g α-Cyclopropyl-(4-chlorphenyl)essigsäurechlorid und 50 ml abs. Toluol werden bei 5-10°C langsam mit 1,27 g Pyridin in 10 ml abs. Toluol versetzt. Nach zwei Stunden bei 20°C wird nochmals zwei Stunden lang bei 50°C ausgerührt. Das Reaktionsgemisch wird 3 mal mit je 20 ml Wasser, einmal mit 20 ml 2n Salzsäurelösung und nocheinmal mit 20 ml Wasser gewaschen und mit Natriumsulfat getrocknet. Nach dem Abdampfen der Lösungsmittel wird das Rohprodukt an Kieselgel mittels Dichlormethan gereinigt.

Man erhält die Verbindung der Formel

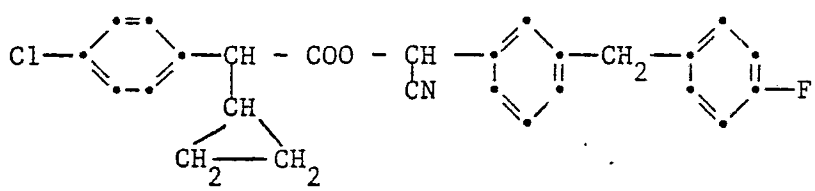

mit einer Refraktion von $n_D^{21°} = 1,5687$

c) Herstellung von α-Prop-1-inyl-3(4-fluorbenzyl)-benzylalkohol

Alkylmagnesiumbromid, hergestellt aus 3,6 g Magnesium und 16,3 g Alkylbromid, in 50 ml Tetrahydrofuran werden bei -25°C zu 6 g Methylacetylen in 20 ml Tetrahydrofuran getropft. Nach dem Erwärmen auf 5°C wird 20 g 3-(4-fluorbenzyl)-benzaldehyd in 20 ml Tetrahydrofuran bei 0 bis 5°C zugetropft und über Nacht bei 20°C ausgerührt. Die Lösung wird mit Sole gewaschen, mit Natriumsulfat getrocknet und eingeengt.

Man erhält die Verbindung der Formel

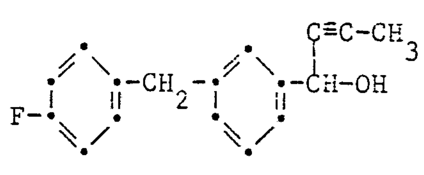

BAD ORIGINAL

mit einer Refraktion von $n_D^{21°} = 1,5611$.

Auf analoge Weise wird auch die Verbindung der Formel

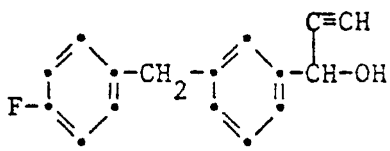

$n_D^{20°} = 1,574$

hergestellt.

d) Herstellung von 3-(4-Fluorbenzyl)-benzaldehyd.

6,5 g 3-(4-fluorbenzyl)-benzylamin in 50 ml Toluol werden bei 10-15°C mit 30 ml frisch destilliertem Isobutyraldehyd versetzt. Nach drei Stunden bei 20°C wird das Wasser abgetrennt, der Ueberschuss an Aldehyd und der Rest des Wassers destillativ entfernt. Das resultierende Oel wird in 50 ml Dimethylsulfat aufgenommen und bei 20°C mit 2 g Kaliumtertiärbutylat versetzt. Nach 1 Stunde bei 20°C wird auf Eis/Wasser gegossen, mit Hexan extrahiert, die Extrakte mit Sole gewaschen, mittels Natriumsulfat getrocknet und die Lösungsmittel destillativ entfernt.
Man erhält die Verbindung der Formel

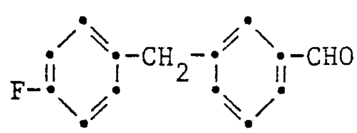

mit einem Siedepunkt von 118°C/0,03 Torr.

BAD ORIGINAL

Patentansprüche

1. Ein 3(Fluorbenzyl)-benzylalkohol der Formel

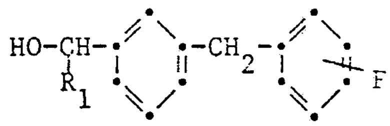

worin $R_1$ Wasserstoff, Cyano, Aethinyl oder Propinyl bedeutet.

2. Eine Verbindung gemäss Anspruch 1 der Formel

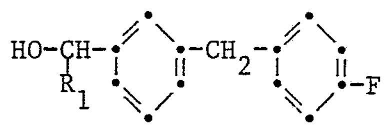

worin $R_1$ Wasserstoff, Cyano, Aethinyl oder Propinyl bedeutet.

3. Die Verbindung gemäss Anspruch 2 der Formel

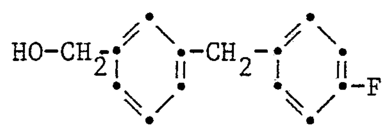

.

4. Die Verbindung gemäss Anspruch 2 der Formel

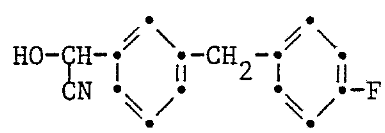

.

5. Die Verbindung gemäss Anspruch 2 der Formel

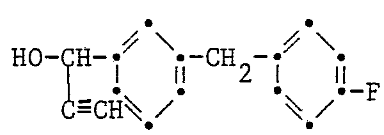

.

6. Die Verbindung gemäss Anspruch 2 der Formel

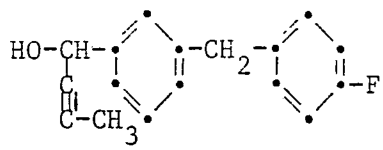

.

7. Verwendung eines Alkohols gemäss Anspruch 1 zur Herstellung von Verbindungen, welche sich zur Bekämpfung von Schädlingen, insbesondere von Insekten oder Vertretern der Ordnung Akarina eignen.

8. Verfahren zur Herstellung eines Alkohols gemäss Anspruch 1, dadurch gekennzeichnet, dass man

a) eine Verbindung der Formeln $CH_3-C{\equiv}C-Mg$ Hal oder $CH{\equiv}C-Mg$ Hal, worin Hal für ein Halogenatom steht, mit der Verbindung der Formel

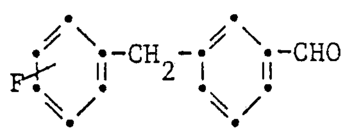

umsetzt, oder

b) die Verbindung der Formel

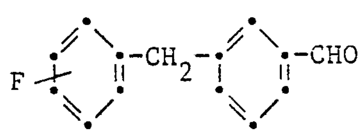

reduziert, oder

c) die Verbindung der Formel

0018315

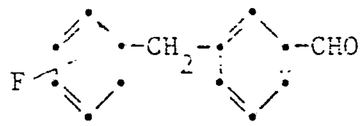

mit der Verbindung der Formel HCN umsetzt.

9. Eine Verbindung der Formel

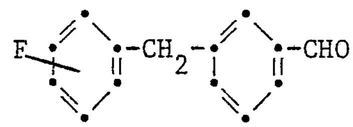

10. Die Verbindung gemäss Anspruch 9 der Formel

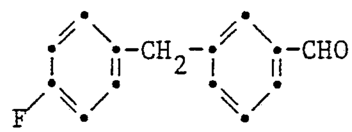
.

Europäisches Patentamt

# EUROPÄISCHER RECHERCHENBERICHT

0018315

Nummer der Anmeldung

EP 80 81 0052.3

| Kategorie | EINSCHLÄGIGE DOKUMENTE — Kennzeichnung des Dokuments mit Angabe, soweit erforderlich, der maßgeblichen Teile | betrifft Anspruch |
|---|---|---|
| | DE - A - 2 231 312 (SUMITOMO CHEMICAL CO.) * Anspruch 11 * | 4 |
| | Chemical Abstracts Band 57, Nr. 10. 12. November 1962 Columbus, Ohio, USA NG. PH. BUU-HOI et al. "Alkylfluorophenylcarbinols with Choleretic Activity" Spalte 12360 a/b & J. Org. Chem. Band 27, 1962, Seiten 2669 bis 2670 | 8 |
| A | DE - A - 2 019 072 (UNIVERSAL OIL PRODUCTS) * Anspruch 1 * | 8 |
| A | US - A - 3 509 180 (M. ELLIOTT) * Spalten 5/6, Zeile 10 * | 1-3 |
| A | US - A - 4 046 799 (V. KAMESWARAN et al.) * Spalte 2, Formel III * | 1-6 |

KLASSIFIKATION DER ANMELDUNG (Int.Cl.3)

C 07 C 33/46
C 07 C 33/48
C 07 C 47/55
C 07 C 121/36
C 07 C 29/00
C 07 C 29/14
C 07 C 29/42
A 01 N 53/00

RECHERCHIERTE SACHGEBIETE (Int. Cl.3)

A 01 N 53/00
C 07 C 29/00
C 07 C 29/14
C 07 C 29/42
C 07 C 31/34
C 07 C 33/042
C 07 C 33/18
C 07 C 33/46
C 07 C 33/48
C 07 C 47/55
C 07 C 121/34
C 07 C 121/36
C 07 C 121/75

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 30-06-1980 | KNAACK |